# EUROPÄISCHE PATENTSCHRIFT

(11) **EP 2 770 047 B1**
(45) Veröffentlichungstag und Bekanntmachung des Hinweises auf die Patenterteilung: **22.04.2015**
(21) Anmeldenummer: 13156779.4
(22) Anmeldetag: 26.02.2013
(51) Int. Cl.: C12M 1/00

(54) **Vorrichtung zum Bereitstellen eines Gases**
Device for providing a gas
Dispositif destiné à la mise à disposition d'un gaz

(43) Veröffentlichungstag der Anmeldung: 27.08.2014
(73) Patentinhaber: ibidi GmbH, 82152 Martinsried (DE)
(72) Erfinder: Öffner, Wolfgang, 68723 Plankstadt (DE)
(74) Vertreter: Grünecker Patent- und Rechtsanwälte PartG mbB

(56) Entgegenhaltungen:
- EP-A1- 1 653 269
- EP-A1- 2 359 870
- EP-A1- 2 492 605
- WO-A1-2012/093326
- DE-C1- 3 815 528
- US-A- 5 025 619

## Beschreibung

Die vorliegende Anmeldung betrifft eine Vorrichtung zum Bereitstellen eines Gases zum Einleiten in eine Inkubationskammer.

Wenn man beispielsweise lebende Zellen in einem Medium über einen längeren Zeitraum inkubieren möchte, ist es wichtig, die Atmosphärenparameter in der Inkubationskammer genau einstellen zu können. Beispielsweise werden Zellen häufig in kleinen Behältern kultiviert, die mit wässrigem Medium gefüllt sind. Eine Verdunstung des wässrigen Mediums führt zu Konzentrationsänderungen in dem Medium, was wiederum zum Absterben der Zellen führen kann. Um diese Verdunstung und Austrocknung zu verhindern, benötigt man daher eine Möglichkeit, die Feuchtigkeit zu regeln, insbesondere eine hohe relative Gas- bzw. Luftfeuchte einzustellen.

Im Stand der Technik sind unterschiedliche Verfahren bekannt, um diese Feuchtigkeitsregelung zu erzielen.

Gemäß einer ersten Alternative werden Vernebler eingesetzt, die in das Gasvolumen zerstäubte Flüssigkeit einsprühen. Die kleinen Wassertropfen werden von dem mit Wasser ungesättigten Gas aufgenommen. Die Gasfeuchtigkeit wird über die Menge an eingesprühter Flüssigkeit geregelt. Eine Reduktion der Feuchtigkeit des Gases kann nur durch Gasaustausch zur Umgebung erzielt werden.

Ein Nachteil eines Verneblers liegt jedoch darin, dass dieser am Düsenausgang ein übersättigtes Gasgemisch aufweist, wodurch es zu Kondensation und zu Korrosion kommen kann. Außerdem muss die Feuchtigkeit des Gasvolumens unter 100 % betragen, um die eingesprühte Wassermenge aufnehmen zu können. Folglich kommt es in dem Gasvolumen zu Bereichen mit relativen Feuchtigkeiten von unter 100 % und in der Nähe der Düse zu einem übersättigten Gas. Solche Gradienten im Gasvolumen sind jedoch nicht wünschenswert. Um die Feuchtigkeit abzubauen, ist außerdem ein Öffnen der Inkubationskammer erforderlich. Schließlich kühlt das Gas am Vernebler wegen der Verdunstungsenergie stark ab. Dies führt zu ebenfalls unerwünschten Temperaturgradienten im Gas.

Eine alternative Lösung stellt ein Verdampfer dar, der üblicherweise einen Behälter mit Wasser umfasst, der im Gasvolumen aufgestellt wird. Die Flüssigkeit im Behälter verdampft und feuchtet das Gas an, bis sich ein Gleichgewicht eingestellt hat. Dieses Gleichgewicht entspricht der Sättigungskonzentration. Solange die Wassermenge ausreichend ist und die Wasseroberfläche entsprechend groß ist, kann auf diese Weise immer ein Gleichgewicht erreicht werden.

Ein Nachteil ist jedoch die Trägheit dieses Systems, da dieses eine gewisse Zeitspanne benötigt, um ins Gleichgewicht zu kommen. Zwar kann die Verdampfung der Flüssigkeit mit einer Temperaturerhöhung beschleunigt werden, jedoch ist auch dieser Prozess nicht sehr schnell. Wird ein System verwendet, das einen Gasaustausch erfordert, ist das Erreichen der Sättigungskonzentration nicht mehr gesichert. Schließlich ist auch die offene Wasseroberfläche im Gasvolumen nachteilig, beispielsweise bei bewegten oder erschütterungsreichen Anwendungen.

Aus der EP 2 492 605 A1 ist ein Verfahren zum Regeln der Temperatur/Feuchtigkeit eines Gases bekannt. Dabei wird basierend auf einer gewünschten Temperatur und einer gewünschten relativen Feuchtigkeit eine Zwischentemperatur berechnet. Eingebrachtes Gas wird mit Wasserdampf vermischt, um die Temperatur des Gases auf die Zwischentemperatur zu erhöhen und die relative Feuchtigkeit des Gases auf 100 % zu erhöhen. Daraufhin wird das Gas auf die gewünschte Temperatur erhitzt, während die Wasserdampfmenge beibehalten wird, so dass die relative Feuchtigkeit auf den gewünschten Wert eingestellt wird.

Schließlich ist auch bekannt, Gaswaschflaschen zu verwenden, um Gas anzufechten. Dabei wird trockenes Gas durch eine Flüssigkeit geleitet, wodurch das Gas angefeuchtet wird. Dieses Anfeuchten passiert schnell, da Gasbläschen eine große Oberfläche mit dem Wasser bilden. Das feuchte Gasgemisch kann dann in das eingeschlossene Gasvolumen der Inkubationskammer geleitet werden. Die relative Feuchtigkeit in dem Gasvolumen in der Inkubationskammer kann dann über die im Gas aufgenommene und transportierte Flüssigkeit geregelt werden. Die im Gas aufgenommene Flüssigkeitsmenge kann über die Wasserbadtemperatur gesteuert werden, da die Aufnahmefähigkeit des Gases temperaturabhängig ist. Für eine Reduktion der Feuchtigkeit ist wiederum ein Gasaustausch im Volumen nötig, also ein Öffnen der Inkubationskammer.

Dieses System erlaubt eine schnell Anfeuchtung des Gases. Um die Feuchtigkeit auch bei einem geringen Gasfluss kontrollieren zu können, muss jedoch viel Feuchtigkeit in dem einströmenden Gas transportiert werden. Die geschieht üblicherweise über die Einstellung der Temperatur des Flüssigkeitsbades, durch das das trockene Gas geleitet wird. Dadurch wird das einströmende Gas erwärmt und die Flüssigkeitsaufnahme gesteigert. Viele Flüssigkeiten, wie beispielsweise Wasser, haben jedoch eine hohe Wärmekapazität, wodurch das System recht träge wird, da die Temperatur nicht schnell eingestellt werden kann. Dadurch kann man die Feuchtigkeit des Gasvolumens in der Inkubationskammer nicht schnell genug regeln. Dies führt häufig zu Kondensationsproblemen, wenn das Gasvolumen in der Nähe der Sättigungskonzentration betrieben wird. Außerdem kann eine gewisse Mindestfeuchtigkeit nicht unterschritten werden, da auch bei einem nicht aufgewärmten Flüssigkeitsbad der Gaswasserflasche das einströmende Gas angefeuchtet wird. Der Regelbereich liegt typischerweise zwischen 60 % und 100 % der relativen Gasfeuchte.

Aufgabe der vorliegenden Erfindung ist es somit, eine Vorrichtung bereitzustellen, mit der der Flüssigkeitsanteil eines Gases rasch und flexibel geregelt werden kann. Diese Aufgabe wird durch eine Vorrichtung nach Anspruch 1 gelöst.

Die erfindungsgemäße Vorrichtung zum Bereitstellen eines Gases zum Einleiten in eine Inkubationskammer, umfasst:
einen Einlassanschluss zum Einleiten von einem Gas,
einen Auslassanschluss zum Ausleiten von einem Gas,
einen ersten Gasweg, der den Einlassanschluss mit dem Auslassanschluss verbindet;
einen zweiten Gasweg, der vom ersten Gasweg abzweigt und wieder in den ersten Gasweg mündet;
ein Verteilelement, das so ausgebildet ist, dass ein Anteil des durch den Einlassanschluss eingeleiteten Gases über den zweiten Gasweg leitbar ist; und
ein im zweiten Gasweg angeordnetes Anreicherungselement zum Anreichern des durchströmenden Gases mit einer Flüssigkeit.

Diese Vorrichtung erlaubt es, einen einstellbaren Anteil eines Gases über den zweiten Gasweg zu leiten und durch das Anreicherungselement mit einer Flüssigkeit anzureichern. Dieses angereicherte Gas kann danach mit dem im ersten Gasweg verbleibenden Gas gemischt werden. Dadurch kann ein Gas bereitgestellt werden, das einen einstellbaren Flüssigkeitsanteil aufweist.

Es hat sich gezeigt, dass durch ein solches System die Feuchtigkeit, also der Flüssigkeitsanteil eines Gases sehr schnell geregelt werden kann. Die Regelzeit wird durch die Gasflussrate bestimmt, und nicht wie bei bekannten Systemen aus dem Stand der Technik durch die Trägheit der Flüssigkeitsbadtemperaturregelung. Außerdem kann die Feuchtigkeit zwischen 0 % und 100 % geregelt werden, indem ein entsprechender Anteil über den zweiten Gasweg geleitet wird.

Bei der Flüssigkeit, mit der das Gas angereichert werden soll, kann es sich insbesondere um Wasser handeln. In diesem Fall soll der Wasserdampfanteil im Gas gesteuert oder geregelt werden. Es ist jedoch auch denkbar, als Flüssigkeit Ethanol oder Toluol zu verwenden. Auch kann die Flüssigkeit eine Kombination mehrerer Flüssigkeiten sein.

Bei dem Gas kann es sich insbesondere um Luft handeln, also um gegebenenfalls komprimierte Atmosphärenluft. Anstelle von Luft könnte auch ein reaktionsarmes Gas, beispielsweise Stickstoff oder Argon, verwendet werden oder ein reaktionsfreudiges Gas, beispielsweise Sauerstoff. Auch kann das Gas eine beliebige Mischung unterschiedlicher Gase sein.

Die Vorrichtung kann insbesondere dem Bereitstellen eines Gases mit einem einstellbaren Flüssigkeitsanteil dienen.

Unter einem Gas mit einem bestimmten Flüssigkeitsanteil kann ein Gas mit einer bestimmten Gasfeuchte oder Feuchtigkeit verstanden werden. Die Flüssigkeit ist dabei in Form eines Flüssigkeitsdampfes, beispielsweise als Wasserdampf, im Gas vorhanden. Die Gasfeuchte kann als relative Gasfeuchte angegeben werden, also als Verhältnis der tatsächlich enthaltenen zur maximal möglichen Flüssigkeitsmasse in einem bestimmten Gasvolumen. Im Fall von Wasser als Flüssigkeit und Luft als Gas kann insbesondere von einer Luftfeuchtigkeit gesprochen werden, die insbesondere als relative Luftfeuchtigkeit angegeben werden kann.

Der Einlassanschluss der Vorrichtung kann insbesondere zum Einleiten eines trockenen Gases vorgesehen sein. Als trockenes Gas wird dabei ein Gas verstanden, das im Wesentlichen keinen Flüssigkeitsanteil aufweist. Dessen relative Gasfeuchte beträgt also 0 %.

Als Gasweg wird hierin insbesondere eine fluidische Verbindung zwischen zwei Punkten bezeichnet, also ein Pfad, auf dem prinzipiell ein Gas strömen kann. Ein Gasweg kann beispielsweise durch eine Gasleitung, beispielsweise in Form eines Rohres oder Schlauches, gebildet oder definiert werden.

Bei dem zweiten Gasweg kann es sich insbesondere um eine Bypassleitung zum ersten Gasweg handeln. Durch den zweiten Gasweg kann es also möglich sein, wenigstens einen Teil des eingeleiteten Gases an wenigstens einem Teil des ersten Gaswegs vorbeizuleiten.

Der zweite Gasweg kann also an einer ersten Stelle vom ersten Gasweg abzweigen und an einer zweite, unterschiedlichen Stelle wieder in den ersten Gasweg münden.

Der Anteil des Gases, der über den zweiten Gasweg leitbar ist, kann insbesondere einstellbar sein. Insbesondere kann das Verteilelement so ausgebildet sein, dass der über den zweiten Gasweg leitbare Anteil einstellbar ist. Der Anteil kann dabei zwischen 0 % und 100 % einstellbar sein. Mit anderen Worten kann das Verteilelement so ausgebildet sein, dass in einer Einstellung das gesamte über den Einlassanschluss eingeleitete Gas über den ersten Gasweg strömt oder in einer anderen Einstellung das gesamte einströmende Gas über den zweiten Gasweg geleitet wird, oder dass in weiteren Einstellungen ein einstellbarer Teil ungleich 0 über den ersten Gasweg und ein einstellbarer Teil ungleich 0 über den zweiten Gasweg geleitet wird.

Der erste Gasweg kann so ausgebildet sein, dass er vom Einlassanschluss bis zur Mündung des zweiten Gaswegs von einem Gas durchströmbar ist, ohne dass sich dessen Gasfeuchte erhöht. Mit anderen Worten kann im ersten Gasweg zwischen dem Einlassanschluss und der Mündung des zweiten Gaswegs kein Anreicherungselement zum Anreichern des durchströmenden Gases mit einer Flüssigkeit angeordnet sein. Dadurch ist es möglich, das mit Flüssigkeit angereicherte, aus dem zweiten Gasweg in den ersten Gasweg strömende Gas mit einem Gas zu mischen, das eine vorherbestimmte Gasfeuchte aufweist, beispielsweise mit einem trockenen Gas.

Im Bereich der Mündung des zweiten Gaswegs in den ersten Gasweg kann eine Mischkammer angeordnet sein, die so ausgebildet ist, dass das Gas aus dem ersten Gasweg und das Gas aus dem zweiten Gasweg gezielt verwirbelt werden. Dadurch ist eine bessere Mischung der Gase möglich.

Der erste Gasweg kann außerdem so ausgebildet sein, dass sich auch zwischen der Mündung des zweiten Gaswegs in den ersten Gasweg und dem Auslassanschluss kein Anreicherungselement zum Anreichern eines Gases mit einer Flüssigkeit befindet. Damit kann die durch die Durchmischung der Gase aus dem zweiten Gasweg und dem vorderen Abschnitt des ersten Gaswegs erhaltene Gasfeuchte konstant gehalten und einer Inkubationskammer zugeführt werden.

Das Verteilelement kann insbesondere steuerbar oder regelbar sein. Mit anderen Worten kann der Anteil des Gases, der über den zweiten Gasweg geleitet wird, steuerbar oder regelbar sein. Dadurch ist es möglich, die Gasfeuchtigkeit zwischen 0 % und 100 % zu regeln. Insbesondere kann die Steuerung oder Regelung auf einer einstellbaren, zu erzielenden Gasfeuchte basieren. Wenn beispielsweise trockenes Gas, also Gas mit Gasfeuchte 0 %, in die Inkubationskammer geleitet werden soll, ist der Anteil des über den zweiten Gasweg zu leitenden Gases 0. Mit anderen Worten ist das Verteilelement so ausgebildet, dass in diesem Fall das gesamte eingeleitete Gas über den ersten Gasweg geleitet wird.

Wird hingegen eine relative Gasfeuchte von 100 % gewünscht, ist der Anteil des über den zweiten Gasweg zu leitenden Gases 100 %. Mit anderen Worten wird das gesamte durch den Einlassanschluss eingeleiteten Gas über den zweiten Gasweg geleitet.

Für dazwischen liegende Gasfeuchten kann der Anteil des über den zweiten Gasweg geleiteten Gases entsprechend bestimmt werden. Die Regelung oder Steuerung des Verteilelements kann mittels eines Steuerungselements der Vorrichtung, beispielsweise in Form eines Mikrocontrollers, geschehen.

Die Vorrichtung kann außerdem einen Sensor zum Bestimmen des Flüssigkeitsanteils eines Gases umfassen, der im ersten Gasweg stromabwärts von der Mündung des zweiten Gaswegs in den ersten Gasweg angeordnet ist oder in einer Inkubationskammer anordenbar ist, wobei das Verteilelement basierend auf einer Messung des Sensors regelbar ist. Insbesondere kann der Anteil des über den zweiten Gasweg geleiteten Gases basierend auf der Feuchtemessung des Sensors regelbar sein. Durch einen derartigen Sensor kann auf einfache Weise eine gewünschte Gasfeuchte eingestellt werden.

Die Vorrichtung kann außerdem einen Temperatursensor zum Bestimmen der Temperatur eines Gases umfassen, der im ersten Gasweg stromabwärts von der Mündung des zweiten Gaswegs in den ersten Gasweg angeordnet ist oder in einer Inkubationskammer anordenbar ist, wobei das Verteilelement basierend auf einer Temperaturmessung des Temperatursensors regelbar ist. Auf diese Weise kann die Regelung weiter verbessert werden, da die relative Gasfeuchte temperaturabhängig ist.

Die oben genannten Sensoren können insbesondere im Gasvolumen der Inkubationskammer anordenbar sein. Es können auch weitere derartige Sensoren zwischen der Mündung des zweiten Gaswegs in den ersten Gasweg und einer Inkubationskammer vorgesehen sein.

Die oben genannten Sensoren können insbesondere mit einer Steuereinrichtung der Vorrichtung verbunden sein, die das Verteilelement steuert oder regelt.

Das Anreicherungselement kann einen Behälter für eine Flüssigkeit umfassen. Die Vorrichtung kann so ausgebildet sein, dass eine im Behälter anordenbare Flüssigkeit von einem über den zweiten Gasweg geleiteten Gas durchströmbar ist. Das Anreicherungselement kann insbesondere wenigstens eine, insbesondere beheizbare, Gaswaschflasche umfassen oder sein. Insbesondere wenn mehrere Flüssigkeiten miteinander kombiniert werden sollen, können mehrere Behälter, insbesondere Gaswaschflaschen, vorgesehen sein, die jeweils mit einer gewünschten Flüssigkeit gefüllt sind. Das Gas kann in diesem Fall im zweiten Gasweg sequenziell die einzelnen Behälter, insbesondere Gaswaschflaschen, durchströmen. Die Behälter können also hintereinander oder in Reihe angeordnet sein.

Das Verteilelement kann insbesondere ein Mischventil sein. Durch ein Mischventil kann der über den zweiten Gasweg geleitete Anteil des eingeleiteten Gases auf einfache Weise gesteuert oder geregelt werden.

Vor und/oder nach dem Verteilelement kann ein Durchflussmesser angeordnet sein. Dieser kann zur Regelung des Verteilelements verwendet werden.

Der Einlassanschluss kann einen Anschluss für wenigstens eine Gasflasche mit vorkomprimierten Gas umfassen. Die Vorrichtung kann alternativ oder zusätzlich einen mit dem Einlassanschluss verbundenen Kompressor und/oder eine mit dem Einlassanschluss verbundene Pumpe umfassen. Dadurch kann ein Gas mit einem vorherbestimmten Druck am Einlassanschluss bereitgestellt werden.

Die Vorrichtung kann außerdem eine Mischvorrichtung zum Mischen unterschiedlicher Gase umfassen, die mit dem Einlassanschluss verbunden ist. In diesem Fall kann das Gas also einem Gasgemisch entsprechen.

Die Mischvorrichtung kann mit mehreren Einrichtungen zum Bereitstellen eines Gases, beispielsweise in Form von Gasflaschen, verbunden sein, wobei jeder Einrichtung zum Bereitstellen eines Gases ein Durchflussventil mit einstellbarer Öffnung zugeordnet ist. Nach wenigstens einem Ventil, insbesondere nach allen Ventilen, kann ein Durchflussmesser positioniert sein. Die von den Einrichtungen zum Bereitstellen eines Gases stammenden Gase können in der Mischvorrichtung zu einem Gasgemisch vermischt werden. Die Mischvorrichtung kann einen Gaskonzentrationssensor umfassen. Über den Gaskonzentrationssensor kann das Mischungsverhältnis der Gase überprüft werden. Basierend auf der Messung des Gaskonzentrationssensors können die Durchflussventile über eine entsprechende rückgekoppelte Regelung geregelt werden, so dass die Konzentrationen im Gasgemisch den erwünschten Konzentrationen entsprechen. Entsprechend kann wenigstens ein Feuchtigkeitssensor zum Regeln der Feuchtigkeit des Gasgemischs vorgesehen sein. In diesem Fall können die Einrichtungen zum Bereitstellen eines Gases so ausgebildet sein, dass sie Gase mit unterschiedlicher Gasfeuchte bereitstellen.

Die oben genannte Pumpe kann insbesondere eine Schwingankerpumpe sein, deren Leistung insbesondere durch Rechteckimpulse variierender Länge geregelt wird. Dadurch ist es möglich, die Elektronik zur Ansteuerung deutlich zu vereinfachen. Die zur Regelung notwendigen digitalen Signale können insbesondere mit einem Zeitgeber eines zur Steuerung des Verteilelements verwendeten Mikrocontrollers erzeugt werden. Die Signale können außerdem mit einer H-Brücke verstärkt werden. Außerdem kann ein Kondensator in Reihe zur Schwingankerpumpe geschaltet werden, der mit der Pumpeninduktivität einen Serienschwingkreis bildet. Auf diese Weise kann die Spannung an der Pumpe einen sinusähnlichen Verlauf aufweisen.

Die Erfindung stellt außerdem ein System umfassend eine oben genannte Vorrichtung und eine Inkubationskammer bereit, wobei die Inkubationskammer über eine Gasleitung mit dem Auslassanschluss der Vorrichtung verbunden ist. Durch ein solches System kann die Gasfeuchte in einem Gasvolumen der Inkubationskammer auf einfache und schnelle Weise geregelt werden. Die Vorrichtung kann insbesondere eines oder mehrere der oben genannten Merkmale aufweisen. Insbesondere können die oben genannten Sensoren in der Inkubationskammer angeordnet sein.

Bei der Inkubationskammer kann es sich insbesondere um eine Inkubationskammer für eine Mikrofluidvorrichtung handeln. Die Inkubationskammer kann also insbesondere eine Inkubationskammer für mikrobiologische Anwendungen, beispielsweise zum Kultivieren von lebenden Zellen, sein.

Die Gasleitung kann wenigstens einen, insbesondere regelbaren, Heizschlauch umfassen. Damit kann die Temperatur und damit die relative Gasfeuchte auf dem Weg von dem Auslassanschluss bis zum Eingang der Inkubationskammer konstant gehalten werden.

Es können in dem System auch mehrere oben beschriebene Vorrichtungen vorgesehen sein, die mit der Inkubationskammer verbunden sind. Diese können in Reihe, also hintereinander, angeordnet sein. Dadurch können auf einfache Weise mehrere Flüssigkeiten miteinander kombiniert werden. Die Vorrichtungen können auch parallel, also unabhängig voneinander, mit der Inkubationskammer verbunden sein. Dadurch können flexibel und schnell unterschiedliche Atmosphären in der Inkubationskammer bereitgestellt werden.

Die Erfindung stellt außerdem ein Verfahren zum Bereitstellen eines Gases zum Einleiten in eine Inkubationskammer, bereit, umfassend die Schritte:
Einleiten von einem Gas in einen ersten Gasweg;
Leiten eines Anteils des Gases über einen zweiten Gasweg, der vom ersten Gasweg abzweigt und wieder in den ersten Gasweg mündet;
Anreichern des Gases im zweiten Gasweg mit einer Flüssigkeit mit Hilfe eines im zweiten Gasweg angeordneten Anreicherungselements, und
Weiterleiten des mit Flüssigkeit angereicherten Gases in den ersten Gasweg.

Durch ein solches Verfahren kann die Gasfeuchte in einem Gasvolumen einer Inkubationskammer schnell und genau geregelt werden.

Das Verfahren kann insbesondere ein Einleiten eines Gases mit einem einstellbaren Flüssigkeitsanteil in die Inkubationskammer umfassen. Mit anderen Worten kann das Verfahren zum Steuern oder Regeln einer Gasfeuchte in einer Inkubationskammer verwendet werden.

Die Flüssigkeit, das Gas, die Inkubationskammer und/oder die Flüssigkeitswege können insbesondere eines oder mehrere der oben genannten Merkmale aufweisen. Insbesondere kann das Verfahren ein Bereitstellen eines oben beschriebenen Systems oder einer oben beschriebenen Vorrichtung umfassen, wobei die Vorrichtung oder das System für die Verfahrensschritte verwendet wird.

Die Erfindung stellt somit auch eine Verwendung eines oben beschriebenen Systems oder einer oben beschriebenen Vorrichtung zum Steuern oder Regeln der Gasfeuchte in einem Gasvolumen einer Inkubationskammer bereit.

Im oben genannten Verfahren kann der Anteil des Gases, der über den zweiten Gasweg geleitet werden soll, zwischen 0 % und 100 % liegen. Der Anteil kann also insbesondere einstellbar sein. Im Fall von 0 % als Anteil werden die Schritte des Leitens des Anteils über den zweiten Gasweg, des Anreicherns des Gases und des Weiterleitens zurück in den ersten Gasweg nicht durchgeführt. Mit anderen Worten, wenn ein Gas mit ursprünglichem Flüssigkeitsanteil, insbesondere trockenes Gas, bereitgestellt werden soll, kann das Verfahren ein Leiten des gesamten Gases von dem Einlassanschluss zu dem Auslassanschluss auf dem ersten Gasweg umfassen.

Im Fall von einem Anteil in Höhe von 100 % wird das Gas nur über den zweiten Gasweg geleitet. In diesem Fall findet kein Mischen mit im ersten Gasweg verbliebenen Gas statt.

Das in den ersten Gasweg eingeleitete Gas kann insbesondere trockenes Gas sein.

Der zweite Gasweg kann insbesondere an einer Stelle vom ersten Gasweg abzweigen und an einer anderen Stelle wieder in den ersten Gasweg münden.

Das Einleiten des Gases in den ersten Gasweg kann ein Regeln eines Eingangsdrucks umfassen. Die Druckregelung kann mit Hilfe einer Schwingankerpumpe stattfinden, wobei die Schwingamplitude und damit die Leistung der Schwingankerpumpe durch Rechteckimpulse variierender Länge geregelt wird.

Das eingeleitete Gas kann insbesondere einem Gasgemisch entsprechen. In diesem Fall kann vor dem Einleiten des Gases ein Mischen von Gasen stattfinden. Dieses Mischen kann mit Hilfe mehrerer Einrichtungen zum Bereitstellen eines Gases durchgeführt werden, wobei jeder Einrichtung zum Bereitstellen eines Gases ein Durchflussventil zugeordnet ist, wobei nach wenigstens einem Ventil, insbesondere nach allen Ventilen, ein Durchflussmesser positioniert ist, der den Durchfluss durch jedes Ventil misst. Die von den Einrichtungen zum Bereitstellen eines Gases stammenden Gase können in einem Mischbereich einer Mischvorrichtung zu einem Gas oder Gasgemisch vermischt werden. Über einen Gaskonzentrationssensor kann das Mischungsverhältnis der Gase überprüft werden. Basierend auf der Messung des Gaskonzentrationssensors können die Durchflussventile über eine entsprechende rückgekoppelte Regelung geregelt werden, so dass die Konzentrationen im Gasgemisch den erwünschten Konzentrationen entsprechen.

Weitere Merkmale und Vorteile der Erfindung werden nachfolgend anhand der beispielhaften Figuren erläutert. Dabei zeigt:
- Figur 1: den schematischen Aufbau einer beispielhaften Vorrichtung zum Bereitstellen eines Gases;
- Figur 2: eine Illustration des zeitlichen Verlaufs einer mit Hilfe einer beispielhaften Vorrichtung zum Bereitstellen eines Gases erreichbaren Feuchtigkeitsregelung; und
- Figur 3: eine Illustration einer möglichen Steuerung zum Regeln eines Drucks eines Gases, das an einem Einlassanschluss einer beispielhaften Vorrichtung zum Bereitstellen eines Gases eingeleitet werden soll.

Im Folgenden wird beispielhaft davon ausgegangen, dass es sich bei dem eingeleiteten Gas um eine Mischung aus CO₂ und Luft handelt, die mit Wasser als Flüssigkeit angereichert werden soll. Die Erfindung ist jedoch nicht auf dieses konkrete Beispiel beschränkt. Als Flüssigkeit wäre beispielsweise auch Alkohol oder Toluol denkbar. Anstelle von Luft und CO₂ (Kohlendioxid) könnte auch ein reaktionsarmes Gas oder ein reaktionsfreudiges Gas verwendet werden. Auch beliebige Mischungen von Gasen sind möglich.

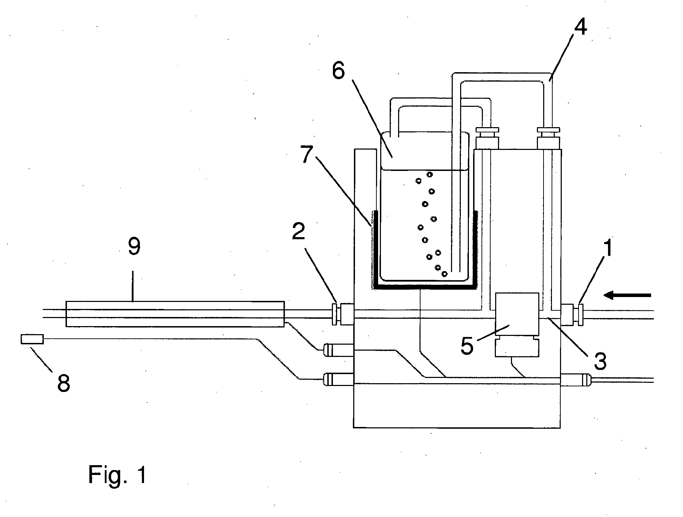

Figur 1 zeigt eine beispielhafte Vorrichtung zum Bereitstellen eines Gases mit einem einstellbaren Flüssigkeitsanteil zum Einleiten in eine Inkubationskammer. Die Vorrichtung umfasst einen Einlassanschluss 1 zum Einleiten von einem Gas, insbesondere einem trockenen Gas, einen Auslassanschluss 2 zum Ausleiten eines Gases und einen ersten Gasweg 3, der den Einlassanschluss 1 mit dem Auslassanschluss 2 verbindet. Ein zweiter Gasweg 4 zweigt an einer ersten Stelle vom ersten Gasweg 3 ab und mündet an einer zweiten, unterschiedlichen Stelle wieder in den ersten Gasweg 3. Mit Hilfe eines Verteilelements 5, das beispielsweise als steuerbares oder regelbares Mischventil ausgebildet sein kann, kann ein einstellbarer Anteil des eingeleiteten Gases über den zweiten Gasweg 4 geleitet werden.

Im zweiten Gasweg ist ein als Gaswaschflasche 6 ausgebildetes Anreicherungselement angeordnet. Dieses kann das durchströmende Gas mit einer Flüssigkeit, in diesem Beispiel mit Wasser, anfeuchten. Die Gaswaschflasche 6 ist über eine regelbare Heizung 7 temperierbar. Als Anreicherungselement sind auch andere Anreicherungselemente denkbar. Es können beispielsweise alternativ oder zusätzlich auch ein oder mehrere Vernebler als Anreicherungselement verwendet werden.

Mit dem Auslassanschluss 2 ist ein regelbarer Heizschlauch 9 verbunden, mit dem das feuchtigkeitsgeregelte Gas zu einer Inkubationskammer (nicht dargestellt) geleitet werden kann. Figur 1 zeigt außerdem einen Temperatur- und/oder Feuchtesensor 8, der beispielsweise in der Inkubationskammer angeordnet sein kann. Basierend auf den Messungen dieses Sensors 8 können beispielsweise das Verteilelement 5, das Heizelement 7 und/oder der Heizschlauch 9 geregelt werden.

Diese Vorrichtung kann beispielsweise für die Feuchtigkeitsregelung in einer Inkubationskammer für Lebendzellenmikroskopie verwendet werden. Dabei kann die Inkubationskammer insbesondere eine Inkubationskammer für Mikrofluidvorrichtungen sein. Die Inkubationskammer kann eine Größe aufweisen, dass genau eine Mikrofluidvorrichtung darin angeordnet werden kann.

In der Anwendung soll ein einströmendes, CO₂-geregeltes trockenes Gasgemisch so angefeuchtet werden, dass die kleinen Wassermengen in den Probenkammern in der Inkubationskammer, in denen die Zellen kultiviert werden, nicht ausdunsten. Nur so können lange Untersuchungszeiten realisiert werden, ohne die Inkubationskammer öffnen zu müssen.

Zunächst wird dazu in einer mit dem Einlassanschluss 1 verbundenen Gasquelle (nicht dargestellt) trockenes Gas, zum Beispiel reines CO₂ und Luft, gemischt und komprimiert. Über den Einlassanschluss 1 wird das Gasgemisch dann in die Vorrichtung geleitet. In der Vorrichtung hat das Gas zwei Wege. Der zweite Gasweg 4 geht durch die Gaswaschflasche 6 mit einem temperierten Wasserbad. Dieser Weg hat einen höheren Durchflusswiderstand. Der andere, erste Gasweg 3 führt an der Gaswaschflasche 6 vorbei und weist einen geringeren Durchflusswiderstand auf.

Mit dem Mischventil 5 kann die Menge an Gas, das an dem Wasserbad vorbeigeführt wird, geregelt werden. Ist das Mischventil 5 komplett geschlossen, wird das gesamte Gas durch die Gaswaschflasche 6 geführt und angefeuchtet. In diesem Fall beträgt die relative Feuchtigkeit des durch die Vorrichtung bereitgestellten Gases 100 % bei Wassertemperatur. Ist das Ventil 5 andererseits komplett offen, so strömt nur trockenes Gas aus der Vorrichtung, da der Gasfluss im ersten Gasweg 3 einen geringeren Widerstand als in dem zweiten Gasweg 4 hat. Je nach Zwischenstellung des Mischventils 5 kann die Feuchtigkeit des Gasgemisches geregelt werden. Der Feuchtesensor 8 in der Inkubationskammer stellt die Rückkoppelung dieses Regelsystems dar.

Die Stellung des Mischventils 5 kann auch zum Optimieren der Wassertemperatur verwendet werden. So kann insbesondere auf einen Temperatursensor im Wasserbad verzichtet werden, und stattdessen das Mischverhältnis der Gase aus den Flüssigkeitswegen 3, 4 analysiert werden. Ist beispielsweise das Wasserbad in der Gaswaschflasche 6 zu kalt, wird kaum trockenes Gas zugemischt. Andererseits, wenn das Wasserbad zu warm ist, wird sehr viel trockenes Gas beigemischt.

Die beschriebene Feuchtigkeitsregelung kann für Inkubationskammern jeglicher Art verwendet werden. Die Inkubationskammer kann insbesondere eine Inkubationskammer für mikrobiologische Anwendungen, beispielsweise zum Kultivieren von lebenden Zellen, sein.

Die beschriebene Feuchtigkeitsregelung hat folgende Vorteile:
Die Feuchtigkeit kann sehr schnell geregelt werden. Die Regelzeit ist nur durch die Gasflussrate bestimmt, nicht mehr durch die Trägheit der Wasserbadtemperaturregelung. Ein schnelles Ventil kann sofort von feuchtem auf trockenes Gas umschalten oder Zwischenstellungen einnehmen.

Die Feuchtigkeit kann zwischen 0 % und 100 % geregelt werden.

Da die Regelung schnell reagiert, kann eine Kondensation in dem Gasvolumen effektiv verhindert werden.

Durch das Verfahren kann mit Gas gearbeitet werden, das Feuchtigkeit bereits aufgenommen hat. Man ist nicht wie im Falle eines aus dem Stand der Technik bekannten Verneblers auf die Flüssigkeitsaufnahme des Gases in dem Gasvolumen der Inkubationskammer angewiesen.

Die Vorrichtung kann außerdem erschütterungsunanfällig aufgebaut werden. Es werden insbesondere keine offenen Flüssigkeitsoberflächen in dem Gasvolumen der Inkubationskammer benötigt.

Die Vorrichtung kann außerdem bei minimaler Gasflussrate betrieben werden, da die Wasserbadtemperatur in der Gaswaschflasche 6 höher gewählt werden kann als im Stand der Technik, wo bei Verwendung einer Gaswaschflasche die Temperatur des Flüssigkeitsbades geregelt wird. Bei höherer Flüssigkeitsbadtemperatur kann in dem feuchten Gas mehr Flüssigkeit transportiert werden. Andererseits kann die Feuchtigkeit auch bei sehr hohen Gasflussraten effektiv geregelt werden.

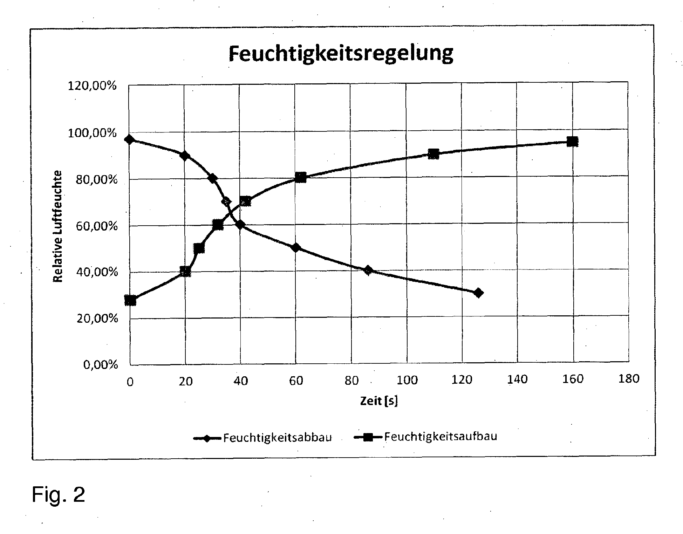

Zur Illustration ist in Figur 2 der experimentell bestimmte Verlauf des Feuchtigkeitsabbaus bzw. Feuchtigkeitsaufbaus mit Hilfe einer oben beschriebenen beispielhaften Vorrichtung gezeigt. Wie aus den Kurven ersichtlich wird, ist es möglich, die relative Luftfeuchte des bereitgestellten Gases innerhalb kürzester Zeit zu regeln. Der Feuchtigkeitsaufbaus ist mit Hilfe von Quadraten, der Flüssigkeitsabbau mit Hilfe von Karos dargestellt.

In Figur 1 wurde ein beispielhaftes System mit nur einer Gaswaschflasche gezeigt. Natürlich könnten auch mehrere Flüssigkeiten kombiniert werden, indem man mehrere hintereinandergeschaltete Gaswaschflaschen verwendet, die jeweils mit einer, gegebenenfalls unterschiedlichen, Flüssigkeit gefüllt sind.

Wie oben ausgeführt, wird über den Einlassanschluss 1 beispielsweise komprimiertes Gas geleitet. Der Druck kann durch vorkomprimierte Gasflaschen, also Druckreservoire, oder durch Kompressoren bzw. Pumpen erzeugt werden. Der Durchfluss kann durch eine feine Druckregelung durch Ansteuerung der Kompressoren bzw. Druckpumpen geregelt werden.

Beispielsweise kann die Vorrichtung eine Schwingankerpumpe umfassen, wobei die Schwingamplitude und damit die Leistung der Pumpe durch Rechteckimpulse variierender Länge geregelt wird. Im Normalfall wird die Pumpleistung von Schwingankerpumpen mit einer sinusförmigen Spannung oder einer Rechteckspannung aus einem Wechselrichter betrieben. Die Leistungssteuerung erfolgt dann mittels Regelventilen oder durch Änderung der Wechselspannungsamplitude. Der Vorteil der hier vorgeschlagenen Rechteckimpulse variierender Länge besteht darin, dass dadurch die Elektronik zur Ansteuerung deutlich vereinfacht werden kann. Insbesondere können die notwendigen digitalen Signale einfach mit einem geeigneten Zeitgeber des ohnehin vorhandenen Mikrocontrollers, der beispielsweise das Verteilerelement 5 oder das Heizelement 7 steuert oder regelt, erzeugt und beispielsweise mit einer H-Brücke verstärkt werden.

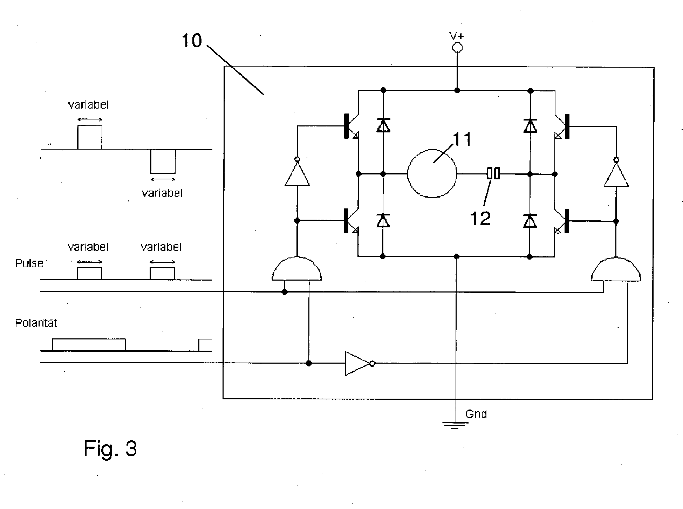

Eine schematische Darstellung einer entsprechenden Regelschaltung ist in Figur 3 dargestellt. Darin ist insbesondere eine integrierte H-Brücke 10 schematisch dargestellt. Integrierte H-Brücken sind am Markt erhältlich, so dass nur wenige Bauteile benötigt werden, um diese Steuerung aufzubauen. Ein Kondensator 12, der in Reihe zur Schwingankerpumpe 11 geschaltet ist, bildet mit der Pumpeninduktivität einen Serienschwingkreis, so dass die Spannung an der Pumpe 11 einen sinusähnlichen Verlauf hat. Die Rechteckimpulse variierender Länge sind in Figur 3 ebenfalls schematisch dargestellt. Insbesondere zeigt Figur 3 die Eingangsimpulse mit entsprechender Polarität und darüber der Spannungsverlauf am Ausgang der H-Brücke:
Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Vorrichtung zum Bereitstellen eines Gases zum Einleiten in eine Inkubationskammer, umfassend:
einen Einlassanschluss (1) zum Einleiten von einem Gas;
einen Auslassanschluss (2) zum Ausleiten von einem Gas;
einen ersten Gasweg (3), der den Einlassanschluss (1) mit dem Auslassanschluss (2) verbindet;
einen zweiten Gasweg (4), der vom ersten Gasweg (3) abzweigt und wieder in den ersten Gasweg (3) mündet;
ein Verteilelement (5), das so ausgebildet ist, dass ein Anteil des durch den Einlassanschluss (1) eingeleiteten Gases über den zweiten Gasweg (4) leitbar ist; und
ein im zweiten Gasweg angeordnetes Anreicherungselement (6) zum Anreichern des durchströmenden Gases mit einer Flüssigkeit;
wobei das Anreicherungselement (6) wenigstens eine, insbesondere beheizbare, Gaswaschflasche und/oder wenigstens einen Vernebler umfasst.

2. Vorrichtung nach Anspruch 1, wobei im ersten Gasweg (3) zwischen dem Einlassanschluss (1) und der Mündung des zweiten Gaswegs (4) kein Anreicherungselement zum Anreichern des durchströmenden Gases mit einer Flüssigkeit angeordnet ist.

3. Vorrichtung nach Anspruch 1 oder 2, wobei das Verteilelement (5) steuerbar oder regelbar ist.

4. Vorrichtung nach einem der vorangegangenen Ansprüche, außerdem umfassend einen Sensor (8) zum Bestimmen des Flüssigkeitsanteils eines Gases, der im ersten Gasweg (3) stromabwärts von der Mündung des zweiten Gaswegs (4) in den ersten Gasweg (3) angeordnet ist oder in einer Inkubationskammer anordenbar ist, und wobei das Verteilelement (5) basierend auf einer Messung des Sensors (8) regelbar ist.

5. Vorrichtung nach einem der vorangegangenen Ansprüche, außerdem umfassend einen Temperatursensor, der im ersten Gasweg (3) stromabwärts von der Mündung des zweiten Gaswegs (4) in den ersten Gasweg (3) angeordnet ist oder in einer Inkubationskammer anordenbar ist, und wobei das Verteilelement (5) basierend auf einer Messung des Sensors regelbar ist.

6. Vorrichtung nach einem der vorangegangenen Ansprüche, wobei das Verteilelement (5) ein Mischventil ist.

7. Vorrichtung nach einem der vorangegangenen Ansprüche, wobei der Einlassanschluss (1) einen Anschluss für wenigstens eine Gasflasche mit vorkomprimierten Gas umfasst oder wobei die Vorrichtung einen mit dem Einlassanschluss (1) verbundenen Kompressor und/oder eine mit dem Einlassanschluss (1) verbundene Pumpe (11) umfasst.

8. Vorrichtung nach Anspruch 7, außerdem umfassend eine Mischvorrichtung zum Mischen unterschiedlicher Gase, die mit dem Einlassanschluss (1) verbunden ist.

9. Vorrichtung nach Anspruch 7 oder 8, wobei die Pumpe (11) eine Schwingankerpumpe ist.

10. System umfassend eine Vorrichtung nach einem der vorangegangenen Ansprüche und eine Inkubationskammer, wobei die Inkubationskammer über eine Gasleitung mit dem Auslassanschluss (2) der Vorrichtung verbunden ist.

11. System nach Anspruch 10, wobei die Gasleitung einen, insbesondere regelbaren, Heizschlauch (9) umfasst.

12. Verfahren zum Bereitstellen eines Gases zum Einleiten in eine Inkubationskammer, umfassend die Schritte:
Einleiten von einem Gas in einen ersten Gasweg (3);
Leiten eines Anteils des Gases über einen zweiten Gasweg (4), der vom ersten Gasweg (3) abzweigt und wieder in den ersten Gasweg (3) mündet;
Anreichern des Gases im zweiten Gasweg (4) mit einer Flüssigkeit mit Hilfe eines im zweiten Gasweg angeordneten Anreicherungselements (6), wobei das Anreicherungselement (6) wenigstens eine, insbesondere beheizbare, Gaswaschflasche und/oder wenigstens einen Vernebler umfasst; und
Weiterleiten des mit Flüssigkeit angereicherten Gases in den ersten Gasweg (3).

## Claims

1. Apparatus for providing a gas for the introduction thereof into an incubation chamber, comprising:
an inlet port (1) for introducing a gas;
an outlet port (2) for discharging a gas;
a first gas path (3) connecting the inlet port (1) to the outlet port (2);
a second gas path (4) branching off from the first gas path (3) and running back into the first gas path (3);
a distributing element (5) configured to allow a portion of the gas introduced through the inlet port (1) to be conducted via the second gas path (4); and
an enriching element (6) arranged in the second gas path for enriching the gas flow with a liquid;
wherein the enriching element (6) comprises at least one, in particular heatable, gas washing bottle and/or at least one atomizer.

2. Apparatus according to claim 1, wherein no enriching element for enriching the gas flow with a liquid is arranged in the first gas path (3) between the inlet port (1) and the orifice of the second gas path (4).

3. Apparatus according to claim 1 or 2, wherein the distributing element (5) is controllable in open-loop or closed-loop.

4. Apparatus according to one of the preceding claims, further comprising a sensor (8) for determining the liquid portion of a gas, which is arranged in the first gas path (3), downstream of the orifice of the second gas path (4) into the first gas path (3), or can be arranged in an incubation chamber, wherein the distributing element (5) can be controlled on the basis of a measurement of the sensor (8).

5. Apparatus according to one of the preceding claims, further comprising a temperature sensor, which is arranged in the first gas path (3), downstream of the orifice of the second gas path (4) into the first gas path (3), or can be arranged in an incubation chamber, and wherein the distributing element (5) can be controlled on the basis of a measurement of the sensor.

6. Apparatus according to one of the preceding claims, wherein the distributing element (5) is a mixing valve.

7. Apparatus according to one of the preceding claims, wherein the inlet port (1) comprises a connection for at least one gas bottle containing a precompressed gas, or wherein the apparatus comprises a compressor connected to the inlet port (1) and/or a pump (11) connected to the inlet port (1).

8. Apparatus according to claim 7, further comprising a mixing device for mixing different gases, which is connected to the inlet port (1).

9. Apparatus according to claim 7 or 8, wherein the pump (11) is an oscillating pump.

10. System comprising an apparatus according to one of the preceding claims and an incubation chamber, wherein the incubation chamber is connected by a gas conduit to the outlet port (2) of the apparatus.

11. System according to claim 10, wherein the gas conduit comprises a, in particular controllable heating hose (9).

12. Method for providing a gas for the introduction thereof into an incubation chamber, comprising the steps of:
introducing a gas into a first gas path (3);
conducting a portion of the gas via a second gas path (4), which branches off from the first gas path (3) and runs back into the first gas path (3);
enriching the gas in the second gas path (4) with a liquid by means of an enriching element (6) arranged in the second gas path, wherein the enriching element (6) comprises at least one, in particular heatable, gas washing bottle and/or at least one atomizer, and
passing the liquid-enriched gas on into the first gas path (3).

## Revendications

1. Dispositif de préparation d'un gaz destiné à être envoyé dans une chambre d'incubation, comprenant :
un raccord d'entrée (1) pour l'introduction d'un gaz ;
un raccord de sortie (2) pour évacuer un gaz ;
un premier parcours de gaz (3), qui relie le raccord d'entrée (1) au raccord de sortie (2) ;
un deuxième parcours de gaz (4), qui est dérivé du premier parcours de gaz (3) et débouche à nouveau dans le premier parcours de gaz (3) ;
un élément de répartition (5), qui est d'une conception telle, qu'une fraction du gaz introduit par le raccord d'entrée (1) peut être envoyée à travers le deuxième parcours de gaz (4) ; et
un élément d'enrichissement (6) agencé dans le deuxième parcours de gaz et destiné à enrichir le gaz qui s'y écoule, avec un liquide ;
l'élément d'enrichissement (6) comprenant au moins une bouteille de lavage de gaz, pouvant notamment être chauffée, et/ou au moins un nébuliseur.

2. Dispositif selon la revendication 1, dans lequel dans le premier parcours de gaz (3), entre le raccord d'entrée (1) et l'embouchure du deuxième parcours de gaz (4), n'est agencé aucun élément d'enrichissement destiné à enrichir le gaz qui s'y écoule, avec un liquide.

3. Dispositif selon la revendication 1 ou la revendication 2, dans lequel l'élément de répartition (5) peut être commandé ou régulé.

4. Dispositif selon l'une des revendications précédentes, comprenant, en outre, un capteur (8) destiné à déterminer la fraction de liquide d'un gaz et agencé dans le premier parcours de gaz (3), en aval de l'embouchure du deuxième parcours de gaz (4) dans le premier parcours de gaz (3), ou pouvant être agencé dans une chambre d'incubation, l'élément de répartition (5) pouvant être régulé en se basant sur une mesure du capteur (8).

5. Dispositif selon l'une des revendications précédentes, comprenant, en outre, un capteur de température agencé dans le premier parcours de gaz (3), en aval de l'embouchure du deuxième parcours de gaz (4) dans le premier parcours de gaz (3), ou pouvant être agencé dans une chambre d'incubation, l'élément de répartition (5) pouvant être régulé en se basant sur une mesure du capteur.

6. Dispositif selon l'une des revendications précédentes, dans lequel l'élément de répartition (5) est une vanne mélangeuse.

7. Dispositif selon l'une des revendications précédentes, dans lequel le raccord d'entrée (1) comprend un raccord de branchement pour au moins une bouteille de gaz renfermant du gaz pré-comprimé, ou le dispositif comprend un compresseur relié au raccord d'entrée (1) et/ou une pompe (11) reliée au raccord d'entrée (1) .

8. Dispositif selon la revendication 7, comprenant, en outre, un dispositif de mélange pour mélanger différents gaz, qui est relié au raccord d'entrée (1).

9. Dispositif selon la revendication 7 ou la revendication 8, dans lequel la pompe (11) est une pompe à induit oscillant.

10. Système comprenant un dispositif selon l'une des revendications précédentes et une chambre d'incubation, système dans lequel la chambre d'incubation est reliée au raccord de sortie (2) du dispositif, par l'intermédiaire d'une conduite de gaz.

11. Système selon la revendication 10, dans lequel la conduite de gaz comprend un tuyau chauffant (9), pouvant notamment être régulé.

12. Procédé pour la préparation d'un gaz destiné à être envoyé dans une chambre d'incubation, comprenant les étapes suivantes :
introduction d'un gaz dans un premier parcours de gaz (3) ;
envoi d'une fraction du gaz à travers un deuxième parcours de gaz (4), qui est dérivé du premier parcours de gaz (3) et débouche à nouveau dans le premier parcours de gaz (3) ;
enrichissement du gaz dans le deuxième parcours de gaz (4), avec un liquide, à l'aide d'un élément d'enrichissement (6) agencé dans le deuxième parcours de gaz, l'élément d'enrichissement (6) comprenant au moins une bouteille de lavage de gaz, pouvant notamment être chauffée, et/ou au moins un nébuliseur ; et
envoi du gaz enrichi en liquide, dans le premier parcours de gaz (3).
